# EUROPÄISCHE PATENTSCHRIFT

(11) **EP 1 206 561 B1**
(45) Veröffentlichungstag und Bekanntmachung des Hinweises auf die Patenterteilung: **19.02.2003**
(21) Anmeldenummer: 00972602.7
(22) Anmeldetag: 27.09.2000
(51) Int. Cl.: C12N 15/82, C12P 21/02, A01H 5/00

(54) **VERFAHREN ZUR HERSTELLUNG PROTEINÖSER SUBSTANZEN**
METHOD FOR PRODUCTION OF PROTEINACEOUS SUBSTANCES
PROCEDE DE PRODUCTION DE SUBSTANCES PROTEIQUES

(30) Priorität: 01.10.1999 DE 19947290
(43) Veröffentlichungstag der Anmeldung: 22.05.2002
(73) Patentinhaber: greenovation Biotech GmbH, 79111 Freiburg i.Br (DE)
(72) Erfinder: RESKI, Ralf, 79254 Oberried (DE); GORR, Gilbert, 79112 Freiburg im Breisgau (DE)
(74) Vertreter: Stürken, Joachim
(86) Internationale Anmeldenummer: DE0003374
(87) Internationale Veröffentlichungsnummer: WO01025456

(56) Entgegenhaltungen:
- WO-A-91/02066
- WO-A-97/04122
- WO-A-98/21348
- WO-A-98/36085
- WO-A-99/38990
- DE-A- 19 629 402
- REUTTER, K., ET AL.: "Production of a heterologous protein in bioreactor cultures of fully differentiated moss plants" PLANT TISSUE CULTURE AND BIOTECHNOLOGY, Bd. 2, Nr. 3, 1996, Seiten 142-147, XP001002564 in der Anmeldung erwähnt
- BORISJUK, N.V., ET AL.: "Production of recombinant proteins form plant root exudates" NATURE BIOTECHNOLOGY, Bd. 17, Mai 1999 (1999-05), Seiten 466-469, XP002169838
- RESKI R: "DEVELOPMENT, GENETICS AND MOLECULAR BIOLOGY OF MOSSES" BOTANICA ACTA,STUTTGART,DE, Bd. 111, Februar 1998 (1998-02), Seiten 1-15, XP000985073 ISSN: 0932-8629
- FIREK S ET AL: "SECRETION OF A FUNCTIONAL SINGLE-CHAIN FV PROTEIN IN TRANSGENIC TOBACCO PLANTS AND CELL SUSPENSION CULTURES" PLANT MOLECULAR BIOLOGY,NL,NIJHOFF PUBLISHERS, DORDRECHT, Bd. 23, 1993, Seiten 861-870, XP002033959 ISSN: 0167-4412
- WEI W S ET AL: "A perfusion air-lift bioreactor for high density plant cell cultivation and secreted protein production" JOURNAL OF BIOTECHNOLOGY,NL,ELSEVIER SCIENCE PUBLISHERS, AMSTERDAM, Bd. 50, Nr. 2, 1. Oktober 1996 (1996-10-01), Seiten 225-233, XP004037059 ISSN: 0168-1656
- DATABASE BIOSIS [Online] BIOSCIENCES INFORMATION SERVICE, PHILADELPHIA, PA, US; Mai 1999 (1999-05) ZEIDLER MATHIAS ET AL: "Transgene expression in the moss Ceratodon purpureus." Database accession no. PREV199900356531 XP002169839 & JOURNAL OF PLANT PHYSIOLOGY, Bd. 154, Nr. 5-6, Mai 1999 (1999-05), Seiten 641-650, ISSN: 0176-1617

## Beschreibung

Die vorliegende Erfindung betrifft allgemein das Gebiet der Herstellung proteinöser Substanzen in pflanzlichen Materialien. Insbesondere betrifft die Erfindung ein neues Verfahren zur Herstellung gewünschter proteinöser Substanzen in Moosen.

Die Verwendung biotechnologischer Verfahren zu Produktionszwekken stellt für den Menschen eine bedeutende Möglichkeit dar, Substanzen zu produzieren, die auf anderem Weg, z.B. durch chemische Synthese, gar nicht bzw. nicht wirtschaftlich herzustellen sind und als Rohstoffe in der Natur nicht in ausreichenden Mengen zur Verfügung stehen. Obwohl mehr als 10.000 Sekundärmetabolite aus Pflanzen bekannt sind, werden nur wenige dieser Verbindungen mit Hilfe von pflanzlichen Zellkulturen in technischen Maßstäben gewonnen. Bei diesen Substanzen handelt es sich in erster Linie um Sekundärmetabolite, die pharmazeutische Wirkungen zeigen. Beispielhaft seien hier a) Berberin (Produktion im 4000 1-Maßstab) mit bakteriostatischer und fungizider Wirkung (Y. Fujita und M. Tabata, in: Plant tissue and cell culture, plant science; Vol. 3, S. 169, C.E. Green et al. (Hrsg.), A. R. Liss Inc., New York (1987)), b) Shikonin (750 1-Maßstab) mit antibiotischer und entzündungshemmender Wirkung (M. Tabata und Y. Fujita, in: Biotechnology in plant science; S. 207-218, P. Day et al. (Hrsg.), Academic Press, Orlando (1985)), und c) Paclitaxel (75000 1-Maßstab), besser bekannt als Taxol, mit Antitumorwirkung (M. Jaziri et al., *Taxus* sp. cell, tissue and organ cultures as alternative sources for taxoids production: a literature survey, Plant Cell Tiss. Org. Cult., **46**, S. 59-75 (1996)) genannt.

Ein weiteres wichtiges biotechnologisches Verfahren, bei dem pflanzliche Zellkulturen genutzt werden, stellt die Biotransformation von Digitoxin in Digoxin, ein Herz- und Kreislaufmedikament, dar. Diese stereospezifische Hydroxylierungsreaktion gelingt mit hohen Ausbeuten in Bioreaktorkulturen von *Digitalis* *lanata* (E. Reinhard und W. Kreis, Kultivierung von pflanzlichen Zellen im Bioreaktor, Bio. Engin., **5**, S. 135-136 (1989)). Eine aktuelle und umfangreiche Übersicht der Verwendung pflanzlicher Zellkulturen in der Biotechnologie findet sich bei H.-P. Mühlbach, Use of plant cell cultures in biotechnology, Biotechnol. Annu. Rev., **4,** S. 113-176 (1998).

Die Entwicklung von genetischen Transformationsmethoden für höhere Pflanzen zu Beginn der 80er Jahre machte es möglich, die Produktivität von Pflanzen für bestimmte sekundäre Inhaltsstoffe durch Transformation der Gene für bestimmte Schlüsselenzyme der entsprechenden Stoffwechselwege deutlich zu erhöhen. Neben der Verwendung transgener vollständiger Pflanzen wurden auch pflanzliche Zellkulturen genutzt. Beispielhaft sei hier die Überexpression einer bakteriellen Lysindecarboxylase in transgenen Wurzelhaarkulturen von *Nicotiana tabacum* genannt, die zu einer Erhöhung der Ausbeuten der biogenen Amine Cadaverin und Anabasin um das bis zu 14-fache führte (J. Berlin et al., Genetic modification of plant secondary metabolism: Alteration of product levels by overexpression of amino acid decarboxylases, in: Advances in Plant Biology, Studies in Plant Science, Vol. **4**, S. 57-81, D.D.Y. Ryu und S. Furasaki (Hrsg.), Elsevier, Amsterdam (1994)).

Durch die Möglichkeit des DNA-Transfers in Pflanzen wurden allerdings nicht nur quantitative und qualitative Veränderungen von Pflanzeninhaltsstoffen möglich. Pflanzen und pflanzliche Zellkulturen wurden darüber hinaus für die Herstellung heterologer Proteine interessant (A.S. Moffat, High-Tech plants promise a bumper crop of new products, Science **256**, S. 770-771 (1992)), wobei im wesentlichen zwei unterschiedliche Ansätze gewählt wurden.

Der eine Ansatz beinhaltet die Produktion heterologer Proteine in transgenen vollständigen Pflanzen. Neben der Produktion von Antikörpern in transgenen Tabakpflanzen (J.K.-C.Ma et al., Generation and assembly of secretory antibodies in plants, Science **268**, S. 716-719 (1995)) wurde die Expression und richtige Prozessierung von humanem Serumalbumin sowohl in transgenen Tabak- als auch in Kartoffelpflanzen beschrieben (P.C. Sijmons et al., Production of correctly processed human serum albumin in transgenic plants, Bio/Techn., **8,** S. 217-221 (1990)). Ebenfalls in transgenen Tabakpflanzen wurde der humane epidermale Wachstumsfaktor (hEGF) exprimiert (A.-H. Salmanian et al., Synthesis and expression of the gene for human epidermal growth factor in transgenic potato plants, Biotechnol. Lett., 18, S. 1095-1098 (1996)). Aber auch andere Pflanzen wurden verwendet. Die Produktion von Leu-Enkephalin wurde erfolgreich mit *Arabidopsis thaliana* und *Brassica napus* durchgeführt (E. Krebbers und J. Vandekerckhove, Production of peptides in plant seeds, Tibtech., **8**, S. 1-3. (1990)). Ferner wurden transgene *Vigna unguiculata* Pflanzen für die Expression von Chimären Viruspartikeln, die als Vaccine dienen, verwendet (K. Dalsgaard et al., Plant-derived vaccine protects target animals against a viral disease, Nat. Biotech., 15, S. 248-252 (1997)).

Ein grundsätzlicher Nachteil bei der Verwendung vollständiger Pflanzen wie der oben beispielhaft beschriebenen liegt in der Notwendigkeit ihrer zeitaufwendigen und kostenintensiven Kultivierung sowie in der für industrielle Produktionsmaßstäbe erforderlichen großdimensionierten Anbaufläche. Darüberhinaus erfordert die Aufreinigung der gewünschten Zielsubstanzen aus vollständigen Pflanzen in der Regel komplexe Verfahrensschritte, insbesondere dann, wenn an die Beschaffenheit und Qualität der Produkte hohe Anforderungen gestellt werden, wie es bei pharmazeutisch oder ernährungsphysiologisch einzusetzenden Substanzen der Fall ist.

Im zweiten Ansatz wurden transgene Tabakzellkulturen für die Produktion von Antikörpern genutzt. Beschrieben ist beispielsweise die Expression von Antikörpern und deren Sekretion ins Medium (N.S. Magnuson et al., Enhanced recovery of a secreted mammalian protein from suspension culture of genetically modified tobacco cells, Prot. Expr. Pur., 7, S. 220-228 (1996)). Da die Aufreinigung heterologer Proteine aus Zellen einen hohen Aufwand erfordert, stellt die Sekretion des Zielproteins in das Medium eine deutliche Verbesserung dar. Darüber hinaus sprechen auch Sicherheitsaspekte für die Produktion rekombinanter, pharmazeutisch relevanter Proteine in Zellkulturen, da die transgenen Pflanzenzellen ausschließlich in Bioreaktoren kultiviert werden können und nicht freigesetzt werden müssen. Die Entwicklung von Bioreaktoren für heterotrophe pflanzliche Zellkulturen in größeren Maßstäben (z.B. M.L. Shuler et al., Bioreactor engineering as an enabling technology to tap biodiversity: The case of taxol., Ann. N. Y. Acad. Sci., **745,** S. 455-461 (1994)) machte die notwendige Massenkultur möglich.

Die grundsätzlichen Nachteile dieses zweiten Ansatzes unter Verwendung pflanzlicher Suspensionskulturen liegen in der geringen Wachtumsrate, der relativ langsamen Bildung von Sekundärmetaboliten, der Hemmung der Produktbildung bei hohen Zelldichten mit der Folge einer geringen volumetrischen Produktivität, der Bildung von Aggregaten und Zellwandbestandteilen, sowie in der erhöhten Sensitivität der Zellen gegenüber Scherkräften. Ferner ist zu berücksichtigen, daß bei Verwendung heterotropher Zellkulturen stets komplexe Medien mit einer Vielzahl z.T. teurer Inhaltsstoffe bereitgestellt werden müssen. Als gravierendster Nachteil ist jedoch das Auftreten somaklonaler Variationen in pflanzlichen *in vitro* Zellkulturen zu nennen, was zu quantitativen und qualitativen Veränderungen in der Produktion heterologer Proteine führt (s. z.B. M.G.K. Jones und K. Lindsey, Plant biotechnology, in: Molecular biology and biotechnology, J.M. Walker und E.W. Gingold (Hrsg.), 2.Aufl., Royal Soc. of Chem., Burlington House, London (1988)). Insbesondere im Zusammenhang mit der Herstellung von Pharmazeutika und anderen gewünschten Substanzen, deren behördliche Zulassung eine verläßliche Qualitätssicherung sowie ein standardisiertes Herstellungsverfahren fordert, ist eine Heterogenität der gebildeten Produkte und ihrer funktionellen Eigenschaften nicht hinnehmbar.

Die Aufgabe der vorliegenden Erfindung liegt daher in der Bereitstellung eines Verfahrens zur standardisierten Herstellung heterologer proteinöser Substanzen in pflanzlichen Materialien, mit welchem sowohl die beschriebenen Nachteile der Verwendung vollständiger Pflanzen als auch die Nachteile der Verwendung von Zellkultursystemen im wesentlichen beseitigt werden.

Erfindungsgemäß wird diese Aufgabe gelöst durch die Bereitstellung eines neuen Verfahrens zur Herstellung heterologer proteinöser Substanzen in pflanzlichen Materialien, bei dem vollständig differenzierte Moospflanzen unter Standardbedingungen kultiviert werden und die Gewinnung der hergestellten proteinösen Substanzen aus dem Kulturmedium im wesentlichen ohne Aufbrechen der produzierenden Gewebe oder Zellen erfolgt.

Der vorliegend verwendete Begriff "proteinöse Substanz" umfaßt Peptide, Polypeptide sowie Proteine als auch Fragmente derselben, welche insbesondere für diagnostische, klinische, pharmazeutische und ernährungsphysiologische Zwecke geeignet sind. Umfaßt sind ferner solche Moleküle, die über peptidische Bindungen verfügen und von pflanzlichem Material translatiert werden.

Nach einer bevorzugten Ausführungsform der vorliegenden Erfindung wird die gewünschte heterologe proteinöse Substanz in ihrer biologisch aktiven Form in das Kulturmedium freigesetzt.

Im Rahmen der vorliegenden Beschreibung bedeutet der Begriff "biologisch aktiv", daß die mit diesem Attribut versehenen Zielsubstanzen über die für den jeweiligen Verwendungszweck gewünschten oder erforderlichen funktionellen Eigenschaften verfügen. Ist beispielsweise die Herstellung von Antikörpern gewünscht, so ist das produzierte Protein bzw. ein funktionelles Fragment desselben biologisch aktiv, wenn es in der Lage ist, die erwartete spezifische Bindung zum Antigen auszubilden. Für den Fachmann ist klar, daß man hierfür nicht immer das vollständige Protein benötigt sondern nach Epitopen oder niedermolekularen Strukturen suchen kann, welche die beabsichtigte biologische Aktivität bzw. Funktionalität sicherstellen. Ein Enzym ist beispielsweise biologisch aktiv, wenn es in der Lage ist, sein Zielsubstrat umzusetzen.

Nach einer weiteren bevorzugten Ausführungsform der Erfindung wird das pflanzliche Material in Form von vollständigen Moospflanzen in einem Kulturmedium kultiviert, welches im wesentlichen frei von Zuckern, Vitaminen und Phytohormonen bzw. funktionellen Fragmenten derselben ist.

Das erfindungsgemäße Verfahren bietet die Möglichkeit der Kultivierung vollständiger ausdifferenzierter Pflanzen unter standardisierbaren photoautotrophen Bedingungen, d.h. ohne das Erfordernis des Zusatzes von Zuckern, Vitaminen und Phytohormonen und dergleichen, wie es bei den im Stand der Technik bekannten heterotrophen Suspensions-Zellkultursystemen gefordert wird. Aufgrund der Verwendung eines kostengünstigen und einfachen Kulturmediums werden die Schritte zur Gewinnung und Aufreinigung der gewünschten Zielsubstanzen erheblich vereinfacht.

Das im erfindungsgemäßen Verfahren einzusetzende pflanzliche Material ist vorzugsweise eine vollständige Moospflanze, ausgewählt aus der Gruppe bestehend aus Laubmoosen und Lebermoosen, wobei Spezies aus den Gattungen *Physcomitrella, Funaria, Sphag* num und *Ceratodon,* bzw. *Marchantia* und *Sphaerocarpos* besonders bevorzugt eingesetzt werden. Am meisten bevorzugt wird das erfindungsgemäße Verfahren unter Verwendung des Laubmooses *Physcomitrella patens* durchgeführt.

Nach einer weiteren bevorzugten Ausführungsform kodiert das zur Transformation verwendete Nukleinsäurekonstrukt nicht nur die gewünschte proteinöse Substanz sondern auch ein Transit-Peptid für die Freisetzung der Substanz aus der Wirtszelle in das Kulturmedium. Jegliche dem Fachmann bekannte autologe und heterologe Nukleinsäuresequenzen sind erfindungsgemäß einsetzbar und können zur Schaffung einer Expressionskassette zur Transformation des Produzenten-Gewebes Verwendung finden. Besonders bevorzugt ist die Verwendung von Signalpeptiden für das endoplasmatische Retikulum oder den zellulären Transport.

Die zur vorliegenden Erfindung durchgeführten Arbeiten zeigen, daß das für Zellkulturen oben beschriebene Problem der somaklonalen Variation in photoautotrophen Flüssigkulturen von Laubmoosen nicht existiert. Ferner bieten die erfindungsgemäß verwendeten Moose gegenüber anderen Systemen den Vorteil einer klaren Abfolge genau definierter Differenzierungsschritte (Chloronema, Caulonema, Knospen, Gametophoren), die durch Zugabe von Pflanzenhormonen (Indol-3-Essigsäure induziert die Caulonemabildung, Isopentenyl-Adenin die Bildung von Knospen) beeinflußbar sind (s. z.B. N.W. Ashton et al., Analysis of gametophytic development in the moss, *Physcomitrella patens,* using auxin and cytokinin resistant mutants, Planta, **144,** S. 427-435 (1979)). Eine gezielte differenzierungsspezifische Expression heterologer Proteine in Bioreaktorkulturen wird somit ermöglicht, wobei eine sich synchron teilende reine und damit homogene Chloronema-Kultur aufgrund ihrer kontrollierbaren, gleichmäßigen Proteinproduktion im Bioreaktor und ihrer Eignung zur Verwendung hormonabhängiger oder differenzierungsspezificher Promotoren erfindungsgemäß besonders bevorzugt geeignet ist.

Insbesondere für die Produktion von Proteinen, die eine kurze Halbwertszeit haben oder cytotoxische Wirkung besitzen, ist neben einem solchen Expressionssystem erfindungsgemäß auch ein induzierbares Promotorsystem verwendbar, wobei der 1'-Promotor aus *Agrobakterium tumefaciens* besonders bevorzugt verwendet wird.

Die erfindungsgemäß vorgeschlagene Kultivierung von Moosen für die Produktion heterologer Proteine unter wirtschaftlichen Gesichtspunkten kann beispielsweise unter Verwendung von *Physcomitrella* in Größenordnungen von 20 ml über 6 1 bis zu 10 l Volumina oder mehr in Schüttelkulturen oder mit Luft begasten Glasgefäßen erfolgen (s. z.B. R. Reski, Zell- und molekularbiologische Untersuchungen der cytokinin-induzierbaren Gewebedifferenzierung und Chloroplastenteilung bei *Physcomitrella patens* (Hedw.) B.S.G., Dissertation, Universität Hamburg (1990)). Da es sich hierbei um die Kultur differenzierter, photoautotropher Pflanzen handelt, müssen dem Medium weder Pflanzenhormone, noch Vitame, noch Zucker hinzugefügt werden. Die Kosten sind im Vergleich zu den komplexen Medien, die z.B. für tierische Zellkulturen benötigt werden, um den Faktor 100 geringer. Erfindungsgemäß hat sich gezeigt, daß die Ausbeute an biologisch aktivem heterologen Protein im Kulturmedium in Gegenwart von PVP um das 35fache gesteigert werden kann, weshalb die Verwendung von PVP im Kulturmedium im erfindungsgemäßen Verfahren bevorzugt ist.

Detaillierte Angaben über die Kultivierung weiterer erfindungsgemäß geeigneter Laubmoose wie beispielsweise *Leptobryum pyriforme* und *Sphagnum magellanicum in* Bioreaktoren sind im Stand der Technik beschrieben (s. z.B. E. Wilbert, Biotechnologische Studien zur Massenkultur von Moosen unter besonderer Berücksichtigung des Arachidonsäurestoffwechsels, Dissertation, Universität Mainz (1991); H. Rudolph und S. Rasmussen, Studies on secondary metabolism of Sphagnum cultivated in bioreactors, Crypt. Bot., **3**, S. 67-73 (1992)). Im Rahmen der vorliegenden Erfindung besonders bevorzugt ist die Verwendung von *Physcomitrella*, insbesondere, da alle gängigen molekularbiologischen Techniken für diesen Organismus etabliert sind (Übersicht bei R. Reski, Development, genetics and molecular biology of mosses, Bot. Acta, **111**, S. 1-15 (1998)).

Für die biotechnologische Nutzung von *Physcomitrella* zur Produktion heterologer Proteine wurden geeignete Transformationssysteme entwickelt. Erfolgreiche Transformationen wurden beispielsweise mit der "Particle gun" durch den direkten DNA-Transfer in Protonemagewebe durchgeführt. Ebenfalls erfolgreich war der PEG-vermittelte DNA-Transfer in Moosprotoplasten. Diese Transformationsmethode wurde für *Physcomitrella* mehrfach beschrieben und führt sowohl zu transienten als auch zu stabilen Transformanten (s. z.B. K. Reutter und R. Reski, Production of a heterologous protein in bioreactor cultures of fully differentiated moss plants, Pl. Tissue culture, @ Biotech., **2**, S. 142-147 (1996)).

Obwohl die vorliegende Erfindung grundsätzlich zur Herstellung jedweder proteinöser Substanzen geeignet ist, wird nachfolgend die Herstellung eines pharmazeutisch relevanten Proteins am Beispiel des humanen "Vascular Endothelial Growth Factor" (VEGF) dargestellt.

Der VEGF wurde erstmals von N. Ferrara und W.J. Henzel isoliert (Pituitary follicular cells secrete a novel heparin-binding growth factor specific for vascular endothelial cells, Biochem. Biophys. Res. Commun., **161,** S. 851-858 (1989)) und als Regulationsfaktor für die kontrollierte Angiogenese und Endothelzellteilung unter normalen physiologischen Bedingungen charakterisiert (N. Ferrara et al., The vascular endothelial growth factor family of polypeptides, J. Cell. Biochem., **47**, S. 211-218 (1991)). Sie konnten ebenfalls zeigen, daß dieser Wachstumsfaktor sehr spezifisch auf vaskuläre Endothelzellen wirkt und für andere Zelltypen inaktiv ist. Der VEGF ist ein über Disulfidbrücken verknüpftes homodimeres Glykoprotein. Vier verschiedene Formen des humanen VEGF sind bekannt. Die vier Isoformen sind 121, 165, 189 und 206 Aminosäuren lang und entstehen durch alternatives Spleißen der VEGF-RNA. VEGF₂₀₆ wurde nur in einer fetalen Leber cDNA nachgewiesen, wogegen Transkripte von VEGF₁₂₁, VEGF₁₆₅ und VEGF₁₈₉ in vielen Tumorzellen und Tumorgeweben detektiert werden konnten. Alle VEGF-Isoformen besitzen Leadersequenzen für die Sekretion, aber nur die beiden kleinsten Formen werden effektiv sekretiert (s. z.B. G. Martiny-Baron und D. Marmé, VEGF-mediated tumor angiogenesis: A new target for cancer therapy, Curr. Opin. Biotechnol., **6**, S. 675-680 (1995)).

Sowohl für die Entwicklung und Verbesserung bestehender Tumor-Therapieansätze als auch für die Charakterisierung des VEGF wurden und werden entsprechende Mengen des VEGF benötigt. Zu Beginn der zur vorliegenden Erfindung durchgeführten Arbeiten war ausschließlich die rekombinante Produktion des VEGF mittels des Baculovirus-Expressionssystems in Insektenzellen beschrieben (z.B. B.L. Fiebich et al., Synthesis and assembly of functionally active human vascular endothelial growth factor homodimers in insect cells, Eur. J. Biochem., **211**, S. 19-26 (1993)). Als weitere Produktionsorganismen kamen *Saccharomyces cerevisiae* (S. Kondo et al., The shortest isoform of human vascular endothelial growth factor/vascular permeability factor (VEGF/VPF₁₂₁) produced by *Saccharomyces cerevisiae* promotes both angiogenesis and vascular permeability, Biochim. Biophys. Acta, **1243,** S. 195-202 (1995)), die Hefe *Pichia pastoris* (D. Mohanraj et al., Expression of biologically active human vascular endothelial growth factor in Yeast, Growth factors, **12,** S. 17-27 (1995)) und *Escherichia coli* (G. Siemeister et al., Expression of biologically active isoforms of the tumor angiogenesis factor VEGF in *Escherichia coli*, Biochem. Biophys. Res. Commun., **222,** S. 249-255 (1996)) hinzu. Mit allen rekombinanten Systemen konnte biologisch aktiver VEGF produziert werden. Das *E. coli* Expressionsystem erfordert jedoch einen hohen Aufwand für die Aufreinigung und Rekonstitution des Proteins, da es in "Inclusion-Bodies" verpackt wird.

### Beispiele

### Zusammenfassung

Mit der Etablierung steuerbarer Massenkulturen von *Physcomitrella patens* (Reutter und Reski, a.a.O.) sowie von Methoden des DNA-Transfers in das Laubmoos *Physcomitrella patens* (K. Reutter, Expression heterologer Gene in *Physcomitrella patens* (Hedw.) B.S.G., Dissertation, Universität Hamburg (1994)) waren die Grundvoraussetzungen für eine biotechnologische Nutzung dieser Pflanze geschaffen.

In zunächst durchgeführten Arbeiten wurde anhand von transgenen Physcomitrella-Linien, die aus der Arbeit von Reutter (a.a.O., 1994) stammten, die langfristige Stabilität der Integration gezeigt. Die Expression der beispielhaft hierfür eingesetzten heterologen *npt* II- und gus-Gene konnte auch nach vier Jahren noch nachgewiesen werden.

Die Bioreaktorkultur von *Physcomitrella* wurde optimiert. Es wurde ein Rührer entwickelt, der eine Zerkleinerung der Protonemen bewirkt und somit die erforderliche Homogenität der Kultur bei kontinuierlichen Umdrehungszahlen von 300 - 500 rpm gewährleistet. Hierdurch wurde eine standardisierte Probenentnahme möglich. Gleichzeitig wurde die zugeführte Luft gleichmäßiger in der Flüssigkultur verteilt. Unter diesen Bedingungen konnte gezeigt werden, daß Biomasse- und Proteinentwicklung ohne pH-Regulation von außen gleich verlaufen wie mit pH-Regulation; diese ist somit überraschenderweise nicht notwendig. Es konnte unter semikontinuierlichen Bedingungen eine wöchentliche Biomasseproduktion von 500 mg Trockengewicht bzw. 22 mg Gesamtprotein pro Liter erzielt werden. Das bedeutet eine Steigerung der Biomasseproduktion um das fünffache gegenüber der herkömmlichen 5 1 Glaskolbenkultur. Die Verringerung der Salzkonzentrationen des Knop-Mediums auf ein Zehntel führte zu ähnlichen Werten und somit zu einer Kostenreduktion.

Die Zugabe von 5mM Ammoniumtartrat beschleunigte durch Verkürzung der lag-Phase die Biomasseentwicklung. Mit der Zugabe von Ammoniumtartrat wurden gleichzeitig Kulturen erhalten, die fast ausschließlich Chloronemazellen umfaßten. Mit Hilfe der Durchflußzytometrie konnte für diese Kulturen gezeigt werden, daß die Zellen sich zu nahezu hundert Prozent in der G2/M-Phase des Zellzyklus befanden. Weitere physiologische Untersuchungen mit Auxin sowie Untersuchungen mit den differenzierungsspezifischen Mutanten *cal*112 und *cal*113 bestätigten dieses Ergebnis und führten zu dem Schluß, daß sich Caulonemazellen in der überwiegenden Zeit in der G1/G0-Phase und Chloronemazellen hauptsächlich in der G2/M-Phase befinden.

Mit dem 1'-Promotor aus Agrobakterien wurde ein Promotor auf eine mögliche Induzierbarkeit im Moos untersucht. Das Gen der β-Glucuronidase (gus) diente als Markergen. In transient transformierten Moosprotoplasten (Transformationsrate = 3x10⁻⁴) konnte nach Induktion mit 5 µM Indol-3-Essigsäure die Expression des gus-Gens beobachtet werden. In den Kontrollen konnte eine Expression in keinem Fall beobachtet werden.

Das Gen für die 121 Aminosäuren große Spleißform des humanen vasculären endothelialen Wachstumsfaktors (VEGF₁₂₁) wurde mit Transformationsraten von 0,5x10⁻⁵ und 3,3x10⁻⁶ in *Physcomitrella* transferiert. Hierfür wurde das Gen hinter den konstitutiven 35S-Promotor und in den für Pflanzen geeigneten Transformationsvektor pRT99 kloniert. In einem zweiten Ansatz wurde zusätzlich die für das zugehörige humane ER-Transitpeptid kodierende Sequenz kloniert. Durch Southern-Analysen der erhaltenen stabilen Transformanten konnte die Integration der heterologen DNA nachgewiesen und die Art der Integration beschrieben werden. Northern-Analysen ergaben für diese Transformanten den Nachweis des nptII- und der beiden VEGF-Transkripte. Der Nachweis der Expression des VEGF₁₂₁ in den Mooszellen konnte mit der indirekten Immunfluoreszenz erbracht werden. Mit Hilfe des Konfokalen Laserscanning Mikroskops konnte das Protein eindeutig in den Zellen lokalisiert werden. Diese Untersuchungen ergaben für die Transformanten ohne Transitpeptid, daß das Protein insbesondere im Cytoplasma lokalisiert ist. In den Tranformanten, die zusätzlich das Transitpeptid für das ER enthalten, ist das Protein in den Kernbereichen und in den Spitzenbereichen der apikalen Zellen - Orte mit sehr hohem ER-Anteil - zu finden. Durch die Anwendung von ELISA sowie zweier Funktionalitätsstests auf das aus dem Kulturmedium gewonnene VEGF-Protein konnte die biologische Aktivität des erfindungsgemäß hergestellten heterologen Proteins nachgewiesen werden.

### Material und Methoden:

Die verwendeten Chemikalien wurden, sofern im Text nicht anders erwähnt, in p.a. Qualität von den Firmen Fluka (Neu-Ulm), Merck (Darmstadt), Roth (Karlsruhe), Serva (Heidelberg) sowie Sigma (Deisenhofen) bezogen.

Lösungen wurden in aufbereitetem, pyrogenfreiem Wasser, im weiteren Text als H₂O bezeichnet, aus einer Milli-Q Water System Wasseraufbereitungsanlage (Millipore, Eschborn) angesetzt.

Restriktionsendonukleasen, DNA-modifizierende Enzyme und molekularbiologische Kits wurden von den Firmen AGS (Heidelberg), Amersham (Braunschweig), Applied Biosystems (Weiterstadt), Biometra (Göttingen), Boehringer Mannheim GmbH (Mannheim), Genomed (Bad Oeynhausen), New England Biolabs (Schwalbach/Taunus), Novagen (Madison, Wisconsin, USA), Pharmacia (Freiburg), Qiagen (Hilden) und Stratagene (Heidelberg) bezogen. Sie wurden, soweit nicht anders erwähnt, nach Herstellerangaben verwendet.

### Vektoren und Konstrukte

Das Plasmid pCYTEXP-VEGF₁₂₁ ist ein Derivat von pCYTEXP1 (T.N. Belev et al., A fully modular vector system for the optimization of gene expression in *Escherichia coli,* Plasmid, **26,** S. 147-150 (1991)), in dem die cDNA des humanen VEGF₁₂₁ für die Expression in *E. coli* integriert ist. Die cDNA des VEGF₁₂₁ wird aus pCYTEXP-VEGF₁₂₁ mit den Restriktionsendonucleasen *Nde* I und *Sal I* herausgeschnitten, gereinigt und "blunt end" in die *Sma* I-Schnittstelle von pRT101 (R. Töpfer et al., A set of plant expression vectors for transcriptional and translational fusions, Nucleic Acids Res., **15,** S. 5890 (1987)) zwischen den 35S-Promotor und die Polyadenylierungssequenz des CaMV kloniert. Mit *Hin* dIII wird die so erhaltene Kassette wiederum herausgeschnitten und in die *Hin* dIII Restriktionsschnittstelle des Transformationsvektors pRT99 kloniert. pRT99 verfügt neben einer multiplen Klonierungsstelle über das Neomycinphosphotransferase-Gen unter der Regulation des 35S-Promotors und der dazugehörigen Polyadenylierungssequenz aus dem CaMV (R. Töpfer et al., Versatile cloning vectors for transient gene expression and direct gene transfer in plant cells, Nucleic Acids Res., **16**, S. 8725 (1988)). Dieses Gen vermittelt in stabil transformierten Pflanzen eine Resistenz gegen das Antibiotikum G418. Die Vermehrung der Plasmide erfolgt in dem *Escherichia coli* Stamm DH5α (J. Sambrook et al., Molecular cloning: a laboratory manual, Cold Spring Harbor Laboratory Press, New York (1989)).

Aus dem urspründlich für die Expression des VEGF₁₂₁ in Insektenzellen konstruierten Vektor pVE-121, der zusätzlich zu der VEGF₁₂₁-Sequenz die DNA umfaßt, die für das natürliche Transitpeptid codiert, welches in tierischen Zellsystemen die Sekretion über das endoplasmatische Reticulum in das Medium vermittelt (Fiebich et al., Synthesis and assembly of functionally active human vascular endothelial growth factor homodimers in insect cells, Eur. J. Biochem., **211,** S. 19-26 (1993)), wird die cDNA durch die Restriktionsenzyme *Bam* HI und *Bgl* II herausgeschnitten und über pRT101 in pRT99 kloniert und überprüft.

Das Plasmid pNA201 ist ein Derivat des binären Vektors pBI101 (A.R. Jefferson et al., Assaying chimeric genes in plants: The GUS gene fusion system, Plant Mol. Rep., **5**, S. 387-405 (1987)). Es enthält als Selektionsmarker für Pflanzen das *nptII*-Gen unter dem Nopalinsynthase-Promotor. Das ebenfalls vorhandene *gus*-Gen wird durch den 1'-Promotor aus *Agrobakterium tumefaciens* reguliert. pNA201 eignet sich für die direkte Transformation von *Physcomitrella patens*.

### Antikörper

Es werden zwei verschiedene Antikörper gegen das VEGF-Protein verwendet. Der erste Antikörper ist ein Kaninchen-Anti-VEGF-Antikörper und gegen ein synthetisches Peptid, welches den Aminosäuren 1-20 des nativen humanen VEGF entspricht, gerichtet (Fiebich et al., a.a.O. (1993)). Der zweite Antikörper ist ein monoklonaler, gegen das humane VEGF₁₂₁ Protein gerichteter Maus-Antikörper (R&D Systems, Wiesbaden).

### Pflanzenmaterial

Eingesetzt wird der Wildtypstamm des Laubmooses *Physcomitrella patens* (Hedw.) B.S.G., der aus der Sammlung des Arbeitsbereiches Genetik im Institut für Allgemeine Botanik der Universität Hamburg stammt. Er geht auf den von H.L.K. Whitehouse in Gransden Wood, Huntingdonshire (England) gesammelten Stamm 16/14 zurück, der aus einer Spore subkultiviert wurde.

Der Wildtypstamm wird entweder in Flüssigkultur mit Knop-Medium (R. Reski und W.O. Abel, Induction of budding on chloronemata and caulonemata of the moss, *Physcomitrella patens*, using isopentenyladenine, Planta, **165**, S. 354-358 (1985)) oder auf Knop-Festmedium mit 1% Oxoid-Agar (Unipath, Basingstoke, England) kultiviert. Die Flüssigkultur wird wie bei Reski (a.a.O., 1990) beschrieben durchgeführt.

### Bioreaktorkultur

Zur Massenanzucht wird Pflanzenmaterial in einem 7 1-Doppelwand-Glasrundkolben Bioreaktor (Applikon Biotek, Knüllwald) kultiviert. Abluftkühler, Belüftungsrohr, pH-Elektrode (Conducta, Gerlingen), Temperaturfühler, Rührwerk, Probenentnahmerohr sowie die Zuflüsse für Säure, Lauge und Medium werden bei diesem Bioreaktorsystem durch Bohrungen im Deckel von oben in den Kulturraum eingeführt. Die Kultivierung erfolgt bei 25°C und wird durch ein entsprechend eingestelltes Wasserbad, welches mit dem Doppelmantel verbunden ist, geregelt. Bei den Versuchsdurchgängen, die mit pH-Regelung durchgeführt werden, wird dieser durch die Titrationseinheit konstant auf pH 5,8 gehalten. Temperaturmessung und pH-Regelung werden durch den Biocontroller ADI 1030 (Applikon Biotek, Knüllwald) gewährleistet. Die Rührerdrehzahl kann durch den Motorregler ADI 1012 (Applikon Biotek, Knüllwald) variiert werden. Das Kulturmedium wird konstant mit 1 bar Luft über ein poröses Einblaselement belüftet. Um Keimfreiheit im Kulturgefäß zu gewährleisten, werden alle Zu- und Abluftleitungen mit Sterilfiltern (Midisart, 0,2 µm; Sartorius, Göttingen) versehen. Die Bioreaktorkultur wird in einem Kulturschrank mit Beleuchtung von der Seite (Weißlicht; Osram L 40W/20; max. 100 µmols⁻¹m⁻²) durchgeführt. Das Animpfen der Kulturen erfolgt mit 0,5 bis 1 g FG Pflanzenmaterial pro Liter Bioreaktorkultur unter sterilen Bedingungen. Das Probenentnahmerohr befindet sich auf Höhe des Rührers, wodurch unter Rühren eine gleichmäßige Probenentnahme gewährleistet wird. Kleine Probenmengen (< 100 ml) werden mit einer sterilen Spritze über einen Luer Lock-Anschluß genommen, für die Entnahme großer Probenvolumina wird beispielweise eine Schlauchpumpe Typ 302/3A mit Kopf 501 RI (Sartorius, Göttingen) verwendet.

Durch die Zugabe von 5 mM Ammoniumtartrat zum Knop-Medium werden Chloronemakulturen des Wildtyps erzeugt.

Unter Anwesenheit des Stabilisators Polyvinylpyrrolidon (PVP) im Kulturmedium kann die Ausbeute an freigesetztem biologisch aktiven heterologen Protein deutlich gesteigert werden.

Die während der Protonemaentwicklung stattfindende Differenzierung des Caulonemas kann durch exogene Zugabe von physiologischen Auxinmengen induziert und verstärkt werden, wobei Konzentrationen von z.B. 5 µmol/l Indol-3-essigsäure (IAA) geeignet sind.

Für die Flüssigkultur von Transformanten unter Selektionsdruck werden dem Knop-Medium 50 mg/l des Antibiotikums G418 (Calbiochem, Bad Soden) zugegeben. Hierzu werden die Kulturen alle zehn Tage direkt vor dem Zerkleinern mit sterilen 100 µm Sieben (Wilson Sieves, Nottingham, England) abfiltriert und in mit Selektionsmedium gefüllten Erlenmeyerkolben überführt.

Für Nährsalzversuche wird das Knop-Medium im Verhältnis 1:10 mit H₂O verdünnt.

### Transformation

Als Transformationsmethode wird der PEG-vermittelte direkte DNA-Transfer in Protoplasten nach Reutter und Reski (a.a.O., 1996) gewählt. Bei jeder Transformation werden 50 µg Plasmid-DNA pro 3x10⁵ Protoplasten eingesetzt. Die Regeneration der Protoplasten und die Selektion auf stabile Transformanten erfolgt, soweit nicht anders erwähnt, nach Reutter und Reski (a.a.O., 1996).

### Indirekte Immunfluoreszenz

| | | |
|---|---|---|
| Puffer | MSB | 100 mM PIPES; 5-10 mM EGTA, 5 mM MgSO₄, pH 6,8 |
| | F-MSB | MSB +5% DMSO |
| | E-MSB | MSB + 5% DMSO + 5% Nonidet |
| | W-MSB | MSB 1:2 mit H₂O verdünnt (Waschpuffer) |
| Enzymlösung | | 1% Cellulase, 1% Pektinase, 2% Driselase in MSB, pH |
| | | 5,6 (alle Sigma, Deisenhofen) |

Zur Fixierung der Moosprotonemen werden diese in 1,25% Glutaraldehyd in F-MSB (v/v) für maximal 10 Minuten inkubiert und kurz in W-MSB gewaschen. Anschließend wird mit 2% Paraformaldehyd in MSB (v/v) für 40 min inkubiert und 3x mit W-MSB gewaschen (1x spülen; 2x 5 min waschen).

Die Reduktion freier, nicht auswaschbarer Aldehyde erfolgt durch Zugabe von MSB und einer Spatelspitze festem Borhydrid mit einer Inkubationszeit von 10 min. Das Borhydrid wird durch dreimaliges Waschen mit W-MSB entfernt.

Im nächsten Schritt werden die Zellwände durch die Zugabe der Enzymlösung für 10 min durchlässig gemacht. Die enzymatische Reaktion wird durch eine pH-Änderung (MSB, pH 6,8) abgestoppt. Es wird erneut 3x mit W-MSB gewaschen.

Durch Inkubation mit einer Detergenzlösung über einen Zeitraum von 120 min werden Chlorophylle extrahiert. Die Lösung wird durch dreimaliges Waschen mit W-MSB wieder entfernt.

Nach dieser Vorbereitung können die Moosprotonemen mit dem primären Antikörper (Anti-VEGF; 1/50 verd.) inkubiert werden. Dies erfolgt für 45 min bei 37°C. Nach dreimaligem Waschen mit W-MSB wird der markierte sekundäre Antikörper (Anti-Kaninchen oder Anti-Maus; 1/30 verd.; mit Fluoresceinisothiocyanat (FITC) (Molecular Probes, Leiden, Niederlande) markiert) für 45 min bei 37°C zugegeben. Zusätzlich zu den 3 Waschschritten wie oben, wird einmal mit W-MSB + 0,1 % Triton gewaschen. Anschließend werden die Protonemen in W-MSB aufgenommen und mindestens über Nacht bei 4°C gelagert.

Die Auswertung erfolgt mit Hilfe eines konfokalen Laserscanning-Mikroskops (CLSM) des Typs TCS 4D (Leica Lasertechnik, Heidelberg) und der Software Scanware 5,0 (Leica Lasertechnik, Heidelberg).

Die Proben werden für die Untersuchung mit dem CLSM auf einem Objektträger in die "Mounting-solution" (Dabco, Sigma, Deisenhofen) gebracht. Die Anregung des an den sekundären Antikörper gekoppelten Fluoreszenzfarbstoffes FITC erfolgt mit Hilfe eines Argon-Krypton Lasers bei einer Wellenlänge von 488 nm. Das FITC emittiert das Licht mit einer Wellenlänge von 528 nm.

### ELISA-Test

Die qualitative und quantitative Bestimmung des in entsprechend transformierten Moospflanzen gebildeten VEGF-Proteins im Kulturmedium erfolgt nach herkömmlichen Verfahren mittels ELISA-Test unter Verwendung der oben beschriebenen Antikörper. Eine Menge von 200 µl Kulturmedium wird direkt dem ELISA-Test zugeführt.

### Funktionalitätstests

Die Überprüfung der biologischen Aktivität des rekombinant gebildeten und aus dem Kulturmedium gewonnenem VEGF erfolgt unter Anwendung des 'Mitogenic Assay' (Miyazono et al., Purification and properties of an endothelial cell growth factor from human platelets, J. Biol. Chem., **262,** S. 4098-4103 (1987)) sowie des 'Day-13 chorioallantoic-membrane angiogenesis Assay' (Wilting et al., A morphological study of the rabbit corneal assay, Anat. Embryol., **183,** S. 1167-1174 (1991)). Zuvor wird das Kulturmedium ultrafiltriert, lyophilisiert und anschließend in Puffer resuspendiert. Gewünschtenfalls kann ein weiterer Reinigungsschritt über eine Kationensäule erfolgen.

### Induktion des 1'-Promotors

Die Induzierbarkeit des 1'-Promotors durch Auxin wird mit 5 µmol/l Indol-3-essigsäure (IAA) getestet. 100 µl Protoplastenaliquots eines Transformationsansatzes mit pNA201 werden fünf Tage nach der Transformation in die Vertiefungen einer 96-Loch-Mikrotiterplatte (Nunc, Wiesbaden) überführt. Die Protoplastensuspensionen werden mit IAA (Endkonz. = 5 µM) für fünf Stunden inkubiert. Die Auswertung der Induktionsversuche erfolgt direkt im Anschluß an die Inkubationszeit mit Hilfe des qualitativen β-Glucuronidase-Nachweises.

### Qualitativer Nachweis der β-Glucuronidase

Die β-Glucuronidaseaktivität wird mit einem qualitativen Test bestimmt (A.R. Jefferson, Assaying chimeric genes in plants: The GUS gene fusion system, Plant Mol. Rep., 5, S. 387-405 (1987)).

| | | |
|---|---|---|
| Substratpuffer | 50 mM K₃Fe(CN)₆ | 50 mM Na₂HPO₄ |
| | 50 mM K₄Fe(CN)₆ | 1 % (v/v) Triton X-100 |
| | 50 mM NaH₂PO₄ | 10 mM EDTA, pH 7,0 |
| | 4 mg/ml PVP (MG 10000) | |
| Färbelösung | 12,5 mg 5-Bromo-4-chloro-3-indolyl-glucuronic-acid (Biomol, Hamburg) gelöst in 250 µl N,N-Dimethylformamid / 50 ml Substratpuffer | |

Moosprotonemen und -protoplasten in Knop- bzw. Regenerationsmedium werden in gleichem Volumen Färbelösung bei 37°C bis zu 72 Stunden inkubiert und direkt im Anschluß unter Verwendung eines Mikroskops ausgewertet.

### Ergebnisse

### Homogenität der Bioreaktor-Kultur - Probenentnahme

Bei Zellkulturen ist eine standardisierte Probenentnahme nur aus homogenen Kulturen gewährleistet. Das Wachstum des Protonemas zu langen Zellfäden führt nach längerer Kulturdauer häufig zu Zellaggregaten und somit zur inhomogenen Verteilung des Pflanzenmaterials in den Flüssigkulturen. Zur Vermeidung dieser Aggregatbildung wird in bestimmten Zeitintervallen - im Bioreaktor ab Tag 10 alle zwei Tage und in der Schüttelkultur alle 12 Tage - eine Zerkleinerung der Protonemen durch Verwendung von Rührern/Homogenisatoren mit hohen Umdrehungszahlen erreicht. Um kontinuierliche Bedingungen im Bioreaktor bei gleichzeitig standardisierter Probenentnahme auch über eine lange Kulturdauer zu ermöglichen, empfiehlt sich die Modifikation eines Turbinenrührers mit drei Rührblättern durch Umfunktionieren der Rührblattränder mittels Anschleifen zu Scherblättern. Hierdurch ist es möglich, durch ständiges "Rühren" mit 300 - 500 rpm homogene Bioreaktorkulturen zu fahren.

Die Entwicklung der Biomasse (in TG [mg/l]) in einem Zeitraum von 35 Tagen (840 h) bei 500 rpm ist in den Kontrollkulturen mit dem Turbinenrührer die gleiche wie in Bioreaktorkulturen, die mit dem Scherblattrührer gerührt wurden.

Die Homogenität der Kulturen wird durch den Vergleich von jeweils sechs parallelen Probeentnahmen beurteilt. Als Vergleichsparameter wird das Trockengewicht von Pflanzenmaterial aus 100 ml Probenvolumen bestimmt. Bei Verwendung eines unveränderten Turbinenrührers nimmt die Standardabweichung mit dem Anstieg der Konzentration der Biomasse zu. Das Rühren mit dem Scherblattrührer hat zur Folge, daß die Standardabweichungen der Probenentnahmen gleich gering bleiben. Dies läßt den Schluß zu, daß mit dem modifizierten Rührer über einen Zeitraum von 35 Tagen eine gleichmäßig homogene Kultur erhalten werden kann.

### Untersuchungen zur Induzierbarkeit des 1'-Promotors

In dem Plasmid pNA201 liegt der 1'-Promotor als Kontrollelement vor dem *gus*-Gen. Für Induzierungsversuche im Moos ist der bekannte β-Glucuronidase-Test als Expressionsnachweis geeignet. In Versuchen mit transgenem Tabak wird beobachtet, daß der 1'-Promotor in Gewebe mit hohem Auxingehalt zur Expression der β-Glucuronidase führt, weshalb für diesen Promotor eine Auxinabhängigkeit vermutet wird. Die Induzierbarkeit des 1'-Promotors durch Auxin in *Physcomitrella patens* wird in transient tranformierten Protoplasten untersucht.

Die Transformationsansätze werden mit (5 h) und ohne Inkubation mit 5 µM Indol-3-essigsäure (IAA) dem β-Glucuronidase-Test unterzogen. In den Kontrollen ohne Zugabe von IAA werden bei der mikroskopischen Auswertung in keinem Ansatz blaue Protoplasten gefunden. Die Auswertung der Protoplaten, die mit Auxin inkubiert werden, ergibt dagegen den Nachweis der Expression des *gus-Gens.* Anhand der blauen Protoplasten wird eine Transformationsrate von 3x10⁻⁴ erzielt. Dies ist ein deutlicher Hinweis auf die Induzierbarkeit des 1'-Promotors in transient transformierten Moosprotoplasten durch das Pflanzenhormon Auxin.

### Erstellung der Vektoren für die VEGF-Transformationen

Für die Transformationen der cDNA des VEGF₁₂₁ ohne Leadersequenz, im weiteren VEGFC genannt, und der cDNA des VEGF₁₂₁ mit Leadersequenz, im weiteren VEGFP genannt, in *Physcomitrella* ist es notwendig, die Sequenzen zwischen eine für Pflanzen geeignete Promotor/Terminierungs-Einheit zu klonieren. Hierfür werden der 35S CaMV-Promotor und das dazugehörige Polyadenylierungssignal gewählt. Die entsprechend vorbereiteten cDNA-Sequenzen des VEGF werden in die *Sma* I Restriktionsschnittstelle der multiplen Klonierungsstelle des Vektors pRT101 kloniert.

Mit einem aus dem Endbereich des 35S-Promotors abgeleiteten Primer werden die entstandenen Vektoren (pRT101VEGFC 3 und VEGFP 21) sequenziert und die korrekte Integration zwischen Promotor und Polyadenylierungsstelle überprüft.

Die entstandenen Kassetten werden mit dem Restriktionsenzym *Hin* dIII herausgeschnitten und in den eigentlichen Transformationsvektor pRT99 in die *Hin* dIII Schnittstelle kloniert (pRT99VEGFC 3 und VEGFP 21). Die Orientierung der Kassetten zur NPTII-Kassette kann über eine Restriktion mit *Sma* I und *Hinc* II ermittelt werden. Bei einer Promotor zu Polyadenylierungssignal-Orientierung erhält man ein 5250 (VEGFC) bzw. 5380 bp (VEGFP) großes Fragment, bei umgekehrter Orientierung ein 1100 (VEGFC)/1230 (VEGFP) sowie ein 4150 bp (VEGFC und P) großes Fragment. Die Restriktionsanalysen lassen nur ein 5250/5380 bp-Fragment erkennen, der Einbau der VEGFC/P-Kassetten ist somit in Promotor zu Polyadenylierungssignal- Orientierung zum *nptII*-Gen des pRT99 erfolgt.

### VEGFC-Transformationen in Physcomitrella

Die absolute Transformationsrate für die Transformation des VEGFC-Konstrukt in Wildtyp-Protoplasten beträgt bei konstanter Stabilität der Transformanten nach mehrfachem Wechsel von Knopmedium zu Selektionsmedium 0,5x10⁻⁵.

### Nachweis der Integration des transformierten Plasmids

Der Nachweis der erfolgten Integration ins pflanzliche Genom wird unter Anwendung der Southern-Hybridisierung erbracht.
Als Sonden werden einerseits ein *Nco* I-Fragment des *npt* II-Gens aus pRT99 und andererseits ein *Nde* I/*Sal* I-Fragment des VEGFC aus pCYTEXP-VEGF₁₂₁ verwendet.

Die in der ungespaltenen Gesamt-DNA der Transformanten detektierten Signale belegen die erfolgreiche Integration der Plasmid-DNA in das pflanzliche Genom. Der Erhalt der gesamten 35S-VEGFC-PolyA-Kassette auch nach der Integration wird durch die Restriktion mit Hin dIII untersucht. Mit diesem Restriktionsenzym wird, sofern die Kassette bei der Integration intakt geblieben ist, ein 1100 bp großes Fragment aus der Gesamt-DNA herausgespalten.

Das Hybridisierungsmuster mit der VEGFC-Sonde zeigt für alle Transformanten ein Fragment von 1100 bp. Damit wird der Nachweis der Integration der vollständigen VEGFC-Expressionseinheit, die Voraussetzung für die korrekte Transkription und Expression des VEGF₁₂₁ im Moos ist, erbracht.

### Nachweis der Transkription der heterologen Gene

Die Transkripte der heterologen Gene VEGFC und NPTII aus den Transformanten werden mit dem nichtradioaktiven DIG-Detektionssystem unter Verwendung der VEGF- und der NPT II-Sonden nachgewiesen. Die Größen für die im Fluorogramm detektierten Transkripte liegen mit 760 Nukleotiden für das VEGFC-Transkript und 1100 Nukleotiden für das NPT II-Transkript in der jeweils erwarteten Größenordnung. In der WT-Kontrolle wird erwartungsgemäß keines der beiden heterologen Transkripte detektiert.

### Analyse der Transformanten mit humanem Transitpeptid

Mit dem PEG-vermittelten DNA-Transfer von 50 µg pRT99P 21 Plasmid-DNA pro Transformationsansatz werden Transformanten erzeugt, die auf Selektionsmedium dauerhaft stabil sind. Daraus ergibt sich eine stabile Transformationsrate von 3,3x10⁻⁶.

### Nachweis der Integration des transformierten Plasmids

Der Nachweis der Integration für die zuvor beschriebenen Transformanten mit Transitpeptid wird wie oben dargelegt mit dem Verfahren der Southern-Hybridisierung unter Verwendung der beschriebenen Sonden erbracht und die Hybridisierung von mit *Hin* dIII gespaltener Gesamt-DNA mit der VEGF-Sonde läßt ein 1230 bp großes Fragment erkennen: der Nachweis der Vollständigkeit der integrierten 35S-VEGFP-PolyA-Kassette.

### Nachweis der Transkription der heterologen Gene

Mit dem oben dargelegten Verfahren können sowohl NPTII- als auch VEGFP-Transkripte nachgewiesen werden.

### Nachweis des humanen VEGF₁₂₁ in transgenen Mooszellen mit dem Konfokalen Laserscanning Mikroskop

Bei dieser Methode wird das zu untersuchende Protein direkt in fixierten Zellen markiert. Die Auswertung erfolgt mit dem Konfokalen Laserscanning Mikroskop, mit welchem ein verbessertes Auflösungsvermögen als mit dem normalen Lichtmikroskop erzielt wird.

In den VEGFC-Transformanten sollte das rekombinante VEGF₁₂₁-Protein, wenn es in den Mooszellen nachweisbar ist, im Cytoplasma akkumulieren. In den VEGFP-Transformanten sollte es im ER-System nachzuweisen sein, wenn das Transitpeptid als Signal im Moos funktioniert.

Mit der Methode der indirekten Immunfluoreszenz und einer computergestützten Auswertung mit dem Konfokalen Laserscanning Mikroskop ist es gelungen, die Expression des humanen VEGF₁₂₁ in transgenen Mooszellen nachzuweisen. Darüber hinaus wird gezeigt, daß mit dem dazugehörigen humanen Transitpeptid der VEGF₁₂₁ in transgenem Moos erfolgreich in das endoplasmatische Retikulum transportiert wird.

### Untersuchung des Vorhandenseins von VEGF im Kulturmedium

Ein Aliquot des Kulturmediums in Gegenwart von PVP mit einem Volumen von 200 µl wird mittels ELISA-Test untersucht und zeigt, daß die mit der Expressionskassette einschließlich Transitpeptid-kodierender Sequenz transformierten Moospflanzen in der Lage sind, VEGF in das Medium freizusetzen. Die positiven Ergebnisse lassen auf ein funktionelles VEGF-Protein schließen.

### Untersuchung der biologischen Aktivität

Beide zur Überprüfung der biologischen Aktivität des in das Kulturmedium freigesetzten VEGF-Proteins eingesetzten Tests liefern positive Resultate und belegen, daß erfindungsgemäß hergestelltes VEGF aus dem Kulturmedium mit der erwünschten biologischen Aktivität gewonnen werden kann.

## Patentansprüche

1. Verfahren zur Herstellung heterologer proteinöser Substanzen in pflanzlichen Materialien, **dadurch gekennzeichnet, daß** als pflanzliches Material Protonema-Moosgewebe verwendet wird und die Gewinnung der hergestellten proteinösen Substanzen aus dem Kulturmedium ohne Aufbrechen der produzierenden Gewebe oder Zellen erfolgt.

2. Verfahren nach Anspruch 1, **dadurch gekennzeichnet, daß** die in das Kulturmedium freigesetzte proteinöse Substanz biologisch aktiv ist.

3. Verfahren nach Anspruch 1 oder 2, **dadurch gekennzeichnet, daß** ein Kulturmedium verwendet wird, welches frei von Zuckern, Vitaminen und Phytohormonen bzw. funktionellen Fragmenten derselben ist.

4. Verfahren nach einem der Ansprüche 1 bis 3, **dadurch gekennzeichnet, daß** das Moosgewebe ausgewählt wird aus der Gruppe bestehend aus Laubmoosen und Lebermoosen.

5. Verfahren nach Anspruch 4, **dadurch gekennzeichnet, daß** das Moosgewebe aus Laubmoosen aus der Gruppe bestehend aus *Physcomitrella, Funaria, Sphagnum* und *Ceratodon* ausgewählt wird.

6. Verfahren nach Anspruch 4, **dadurch gekennzeichnet, daß** das Moosgewebe aus Lebermoosen aus der Gruppe bestehend aus *Marchantia* und *Sphaerocarpos* ausgewählt wird.

## Claims

1. A method for the production of heterologous proteinaceous substances in plant material, **characterized in that** protonema moss tissue is used as plant material and that the proteinaceous substances produced are obtained from the culture medium without disrupting the producing tissues or cells.

2. The method according to claim 1, **characterized in that** the proteinaceous substance released into the culture medium is biologically active.

3. The method according to claim 1 or 2, **characterized in that** a culture medium is used which is free from sugars, vitamins and phytohormones or functional fragments thereof.

4. The method according to any of claims 1 to 3, **characterized in that** the moss tissue is selected from the group of the mosses including liverworts.

5. The method according to claim 4, **characterized in that** the moss tissue is selected from mosses of the group consisting of *Physcomitrella, Funaria, Sphagnum* and *Ceratodon.*

6. The method according to claim 4, **characterized in that** the moss tissue is selected from liverworts of the group consisting of *Marchantia* and *Sphaerocarpos.*

## Revendications

1. Procédé de production de substances protéiques hétérologues dans des matériaux végétaux, **caractérisé en ce que** l'on utilise des tissus de protonema de bryophytes en tant que matériau végétal et **en ce que** l'obtention des substances protéiques fabriquées se fait à partir du milieu de culture sans rupture des tissus ou des cellules productrices.

2. Procédé selon la revendication 1, **caractérisé en ce que** la substance protéique libérée dans le milieu de culture est biologiquement active.

3. Procédé selon la revendication 1 ou 2, **caractérisé en ce que** l'on utilise un milieu de culture qui est exempt de sucres, de vitamines et de phytohormones ou de fragments fonctionnels de ces dernières.

4. Procédé selon l'une des revendications 1 à 3, **caractérisé en ce que** le tissu de bryophyte est sélectionné parmi le groupe se composant des mousses et des hépatiques.

5. Procédé selon la revendication 4, **caractérisé en ce que** le tissu de bryophyte est sélectionné parmi les mousses du groupe se composant de *physcomitrella, Funaria, Sphagnum* et *Ceratodon.*

6. Procédé selon la revendication 4, **caractérisé en ce que** le tissu de bryophyte est sélectionné parmi les hépatiques du groupe se composant de *Marchantia* et *Sphaerocarpos.*
